# EUROPEAN PATENT SPECIFICATION

(11) **EP 3 535 590 B1**
(45) Date of publication and mention of the grant of the patent: **03.05.2023**
(21) Application number: 17780768.2
(22) Date of filing: 11.10.2017
(51) Int. Cl.: G01N 33/92, A61L 15/20, G01N 33/52

(54) **MALODOUR SAMPLING METHOD**
VERFAHREN ZUR PROBENAHME VON SCHLECHTEM GERUCH
PROCÉDÉ D'ÉCHANTILLONNAGE DE MAUVAISES ODEURS

(30) Priority: 02.11.2016 EP 16196859; 02.11.2016 EP 16196872
(43) Date of publication of application: 11.09.2019
(73) Proprietor: Unilever IP Holdings B.V., 3013 AL Rotterdam (NL); Unilever Global IP Limited, Wirral, Merseyside CH62 4ZD (GB)
(72) Inventor: BATES, Susan, Bebington Wirral Merseyside CH63 3JW (GB); HADDLETON, David, Mark, Coventry Warwickshire CV4 7AL (GB); HAND, Rachel, Alice, Coventry Warwickshire CV4 7AL (GB); KHOSHDEL, Ezat, Bebington Wirral Merseyside CH63 3JW (GB)
(74) Representative: Whaley, Christopher
(86) International application number: PCT/EP2017/075994
(87) International publication number: WO 2018/082882

(56) References cited:
- WO-A1-00/69405
- WO-A1-02/087645
- WO-A1-2006/111748
- WO-A2-2008/154546
- WO-A2-2009/019485
- US-A- 5 441 048

## Description

### Field of Invention

The present invention is in the field of sweat sampling devices and methods for sampling and analysing volatile malodourous materials present on the skin of the human body.

### Background

The surface of the human body, i.e. the skin, produces sweat from both eccrine and apocrine glands. Eccrine secretions are part of the body's thermoregulatory function and typically occur at elevated temperature and/or during physical exercise. Apocrine secretions are typically produced as a result of emotional and/or physical stress. Both secretions may ultimately result in the production of volatile malodourous materials and methods for sampling and analysing these are required in order to understand and counter the production of these malodourous materials.

Current methods for sampling and analysing volatile malodourous materials present on the skin of the human body are fairly crude. In one method, textile swatches are placed on the inside of panellists' shirts in the underarm area and sweat is absorbed onto these as it is produced in the axillae. The swatches may later be extracted with an appropriate solvent and the resulting solution analysed for the presence of volatile malodourous materials. This method is less than ideal in that the swatches are uncomfortable to wear for the panellists and the capture of sweat is inefficient. In addition, the textile swatches may be contaminated with chemicals involved in their manufacture, which may interfere with the subsequent analysis.

WO 98/027909 A1 (P&G, 1998) and several other publications by this applicant disclose disposable absorbent articles for topical adhesive attachment that can serve as sanitary napkins, pantiliners, adult incontinence products or sweat pads.

US 2012/0114591 A1 (First Water Ltd., 2012) discloses the use topical hydrogel treatment of wounds, the hydrogel comprising a hydrophilic polymer carrying multiple pendant sulphonyl groups.

US 2012/0116279 A1 (First Water Ltd., 2012) discloses the use of a multilayer dressing for wounds, the dressing including a gel layer in contact with the wound.

WO2009/019485 discloses a wound dressing comprising a hydrogel.

WO2008/154546 relates to a patch comprising an odour control agent wherein a hydrogel acts as an adhesive matrix.

WO2006/111748 and US5441048 disclose collecting volatile materials from the skin but lack the presence of a hydrogel.

WO2002/087645 discloses the use of hydrogels for wound dressing, drug delivery and pressure relieving cushioning.

WO2000/6940 discloses the use of a hydrogel in a therapeutic patch for treating skin problems e.g. acne.

### Summary of the Invention

In a first aspect of the invention, there is provided a method of sampling volatile malodorous materials from the human skin, said method comprising the topical application of a patch comprising an absorbent hydrogel layer and a backing sheet adhering to it, the volatile malodourous materials being absorbed into the hydrogel layer and subsequently being extracted from it.

In a second aspect of the invention, there is provided a patch for sampling volatile malodourous materials from the human skin comprising an absorbent hydrogel layer, a backing sheet adhering to it and a permeable inner membrane, which is held between the hydrogel layer and the skin when the patch is in use.

It is an object of the present invention to provide an improved method for sampling volatile malodorous materials from the surface of the human body, i.e. the skin, in particular the underarm regions thereof.

It is a further object of the present invention to provide a comfort-in-use patch for sampling volatile malodourous materials from the human skin, in particular the underarm regions thereof.

It is a further object of the present invention to provide a means for sampling sweat and volatile malodourous materials that does not involve use of a biological substrate for the absorption of the sweat. We have found that such substrates are poor carriers for volatile malodourous materials, being readily susceptible to microbial contamination, which can result in modification to the profile of malodourous volatiles originally present.

Particular benefits of the present invention include comfort in use and efficient collection of perspiration, i.e. sweat.

Other benefits of the present invention include the ability of the patch to absorb sweat and volatile components direct from the skin over short/long periods of time (minutes to hours) without occluding the skin surface, and with no transfer of adhesive to the skin.

In addition, the hydrogels used in accordance with the present invention are able to store and release volatile malodourous materials far more effectively than cotton and other natural absorbents used in the prior art. Benefits such as improved absorption, reduced degradation and improved ease of extraction are possible.

### Detailed Description of the Invention

Herein, all percentages, parts and ratios are to be understood to be by weight, unless otherwise stated.

Herein, all amounts, percentages and ratios are to be understood as prefixed by the word "about".

Herein, any component, ratio or weight indicated as "preferred" is to be understood as preferably used in combination within any other component, ratio or weight indicated as "preferred".

Herein, the terms "comprising" and "comprised of", etc., are to be understood as meaning that other components/features could also be present; i.e. the listed steps or options need not be exhaustive.

Herein, the "inner" part/layer/section of the patch is that part/layer/section closer or closest to the skin when the patch is in use and the "outer" part/layer/section of the patch is that part/layer/section farther or farthest from the skin when the patch is in use.

Herein, "breathable" means that air is able to pass completely through the material at ambient conditions.

Herein, "permeable" means that fluids, in particular water, is able to pass through the material, at least in part, by wicking or by any other means at ambient conditions.

Herein, "ambient conditions" mean 25°C and one (1) atmosphere pressure and ambient conditions are to be understood to be prevalent unless otherwise stated.

Herein, "volatile" means having a significant vapour pressure (e.g. greater than 5 Pa or 10 Pa) at 25°C.

### Hydrogel layer

An absorbent hydrogel layer is an essential component of the present invention. The hydrogel layer needs to be in direct or indirect contact with the skin during the use of the patch in order to allow the volatile malodourous materials present of the skin to be absorbed into the hydrogel layer.

Herein, the term "hydrogel" is not to be considered as limited to gels that contain water, but extend generally to all hydrophilic gels, including those containing organic non-polymeric components.

The hydrogel may be selected from non-ionic, anionic, cationic and zwitterionic natural hydrophilic biopolymers, synthetic hydrophilic polymers, hydrocolloids, gelling hydrophilic biopolymers and all combinations thereof.

Hydrogels are, generally speaking, hydrophilic polymers characterised by their ability to absorb large amounts of aqueous fluid without dissolving in said fluid.

The hydrogel hydrophilicity is generally due to groups such as hydroxyl, carboxyl, carboxamido, and esters. On contact with water, the hydrogel assumes a swollen hydrated state that results from a balance between the dispersing forces acting on hydrated chains and cohesive forces that hold the hydrogel together, but do not prevent the penetration of water into the polymer network. The cohesive forces are most often the result of crosslinking via covalent bonds, but may result from weak interactions such as electrostatic, hydrophobic or dipole-dipole interactions.

The hydrogel layer is a coherent, three-dimensional polymer capable of absorbing water without liquefying, i.e. it is insoluble in water. Usually, the insolubility in water is provided by crosslinking of a "base" polymer, although certain hydrogel are by their nature "crosslinked", for example, when the crosslinking occurs during the biosynthesis of the hydrogel.

Herein, the "base" polymer for the hydrogel layer is to be considered the total polymer in the theoretical absence of any crosslinking agent. In practice, the "base polymer" may never be formed; however, it may still be considered present in the hydrogel as the sum of the non-crosslinking monomers used in the synthesis.

It is particularly important to use a hydrogel layer comprising a crosslinked hydrophilic polymer when the extraction technique is using supercritical fluid, in particular supercritical carbon dioxide and especially supercritical carbon dioxide at a temperature of from 20°C to 50°C and at a pressure of from 100 to 300 Bar (from 10 to 30 MPa). The cross-linked nature of the hydrogel makes it sufficiently robust to survive these particular extraction techniques.

The absorption of the volatile malodourous materials into the hydrogel layer is as part of the perspiration of which they are components and typically occurs by wicking of the perspiration into the hydrogel. This wicking is aided by the hydrogel comprising an hydrophilic polymer, in particular a hydrophilic polymer that is an addition polymer having pendant hydrophilic groups.

Suitable hydrophilic polymers for use as or in the hydrogel layer are gelatin, polysaccharides, crosslinked acrylamide polymers, thermoplastic polyurethanes (TPUs), crosslinked acrylates or methacrylates, crosslinked polymers and copolymers of N-vinylpyrrolidinone and crosslinked polymers and copolymers of acrylic acid.

Particularly suitable crosslinked acrylate and methacrylate hydrogels are ones comprising monomers selected from hydroxyethylacrylate, hydroxyethylmethacrylate, 2-(N,N-dimethylamino)ethyl methacylate and methacryloyloxyalkyl sulfonates (generally crosslinked with di-acrylate or di-vinylbenzene).

Particularly suitable crosslinked acrylamide hydrogels are ones comprising monomers selected from poly(2-acrylamido-2-methylpropane sulfonic acid) and salts thereof.

Hydrogels are described in greater detail in Hydrogels, Kirk-Othmer Encyclopaedia of Chemical Technology, 4th Edition, vol. 7, pages 783-807, John Wiley and Sons, New York.

Preferred hydrophilic polymers for use in the present invention are metal salts of crosslinked of poly(2-acrylo-2-methyl-1-propanesulfonic acid) (PolyAMPS) and poly(2-hydroxyethylmethacrylate). A particularly preferred hydrophilic polymer is crosslinked sodium poly(2-acrylo-2-methyl-1-propanesulfonate). The non-neutralised base monomer and polymer [poly(2-acrylo-2-methyl-1-propanesulfonic acid^{®}] are available from Lubrizol Corp.

An especially preferred hydrogel for use in accordance with the present invention is sodium poly(2-acrylo-2-methyl-1-propanesulfonate) crosslinked with poly(ethylene glycol) diacrylate.

A further hydrophilic polymer that may be used as the hydrogel layer is Tecophilic^{™} TG-2000, which is a TPU, also available from Lubrizol Corp.

Differing techniques may be used to cause cross-linking of the hydrogel layer. Cross-linking may occur through condensation polymerization of monomers having multiple functionality or by covalent bonding between polymeric chain by techniques such irradiation, sulphur vulcanization, or other chemical.

The synthesis of the hydrogel is preferably a free radical polymerisation with cross-linking, which may, for example, be induced by light, heat, radiation (e.g. ionising radiation), or redox catalysis. The precursor solution may include appropriate initiators, as generally known in the art.

Crosslinking may also be carried out by reacting biopolymers or synthetic polymers with di- or multi-functional reactive molecules. For example, taking a polysaccharide or polyvinyl alcohol and reacting it with a reactive molecule such as epichlorohydrin, a polyacid, polyanhydride, or di-isocynates, can produce a cross-lined hydrogel.

Another example of cross-linking is to take a poly(acrylic acid) or carrageenan and crosslink it with polyethylene glycol or a Jeffamine.

Multi-functional monomers, in particular di-functional monomers, are suitable cross-linking agents used in the preparation of the hydrogel layer. Such monomers can impart crosslinking or branching sites to give the hydrogel its 3-dimensional "architecture".

Suitable di-functional cross-linking monomers are 2,2-bis[4-(2-acryloxyethoxy) phenyl] propane, bis(2-methacryloxyethyl)-N,N'-1,9-nonylene biscarbamate, 2,2-bis(4-methacryloxyphenyl) propane, 2,2-bis[4-(2-hydroxy-3-methacryloxy- propoxy)phenyl] propane, 1,4-butanediol diacrylate, 1,3-butanediol dimethacrylate, 1,4-butanediol dimethacrylate, trans-1,4-cyclohexanediol dimethacrylate, N,N'-cystaminebisacrylamide, 1,10-decanediol dimethacrylate, 1,4-diacryloylpiperazine, N,N'-diallylacrylamide, diethylene glycol diacrylate, 2,2-dimethylpropanediol dimethacrylate, dipropylene glycol dimethacrylate, N,N'-ethylene bisacrylamide, ethylene glycol diacrylate, ethylene glycol dimethacrylate, N,N'-hexamethylenebisacrylamide, 1,6-hexanediol diacrylate, N,N'-methylenebisacrylamide, nonanediol dimethacrylate, 1,5-pentanediol dimethacrylate, 1,4-phenylene diacrylate, tetraethylene glycol dimethacrylate, triethylene glycol diacrylate, triethylene glycol dimethacrylate and zinc dimethacrylate.

Suitable tri-and tetra-functional and cross-linking monomers are 1,1,1-trimethylolpropane triacrylate, 1,1,1-trimethylolpropane trimethacrylate, di-pentaerythritol penta-acrylate (mixture of tetra-, penta- and hexa-acrylates) and pentaerythritol tetra-acrylate (mixture of tri- and tetra esters).

A particularly suitable crosslinking agent for the hydrogel layer is methylene-bisacrylamide, when the base polymer of an acrylamide derivative such as 2-acrylamido-2-methylpropanesulfonic acid or one of its salts.

Other particularly suitable crosslinking agent for the hydrogel layer are -bis-acrylates and bis-methacrylates, particularly when the base polymer is an acrylate or methacrylate derivative, for example, poly(2-hydroxyethylmethacrylate).

Generally, the level of crosslinking agent relative to the base polymer is quite low, usually equating to a weight ratio of less than 1:50. Preferably, this ratio is from 1:500 to less than 1:50 and more preferably, it is from 1:400 to 1:100. For the avoidance of doubt, the ratio of crosslinking agent to base polymer should be understood to equate to the ratio of crosslinking monomers to non-crosslinking monomers used in preparation of any such hydrogel.

The hydrophilic polymer part of the hydrogel is general its major component and preferably comprises greater than 80% of said layer. In some preferred embodiments, the hydrophilic polymer is the sole component of the hydrogel layer, other than the crosslinking agent that may be present therein.

In preferred embodiments, the hydrogel layer comprises one or more organic plasticisers that can serve to enhance the flexibility of the hydrogel. Suitable plasticisers may comprise any of the following either alone or in combination: at least one polyhydric alcohol (such as glycerol, polyethylene glycol, or sorbitol), at least one ester derived therefrom, at least one polymeric alcohol (such as polyethylene oxide) and/or at least one mono- or poly-alkylated derivative of a polyhydric or polymeric alcohol (such as alkylated polyethylene glycol). Glycerol is a preferred plasticiser. An alternative preferred plasticiser is the ester derived from boric acid and glycerol. When present, the organic plasticiser may comprise up to about 45% of the hydrogel layer, but is preferably from 1 to 30% and more preferably 1 to 10% thereof.

Any compatible surfactant or combination thereof may advantageously be used as an additional ingredient of the hydrogel layer. Such surfactants can lower the surface tension of the mixture before polymerisation and thus aid processing. The surfactant may be non-ionic, anionic, zwitterionic or cationic. The surfactant may itself be reactive, i.e. capable of participating in the hydrogel-forming reaction. The total amount of surfactant, if present, is suitably up to about 10% by weight of the hydrogel, but is preferably from 0.05% to 4% thereof.

In a preferred embodiment of the invention, the surfactant is at least one propylene oxide/ethylene oxide block copolymer, for example that supplied by BASF Plc under the trade name Pluronic^{®} P65 or L64.

The hydrogel layer typical has a sticky nature/feel. This serves to enhance the adhesion of the hydrogel layer to the skin and is therefore a preferred feature.

The sticky feel can, however, lead to sensory negatives with regard to its contact with the skin, a problem that can be alleviated by the presence of a permeable inner membrane, which is held between the hydrogel layer and the skin when the patch is in use (*vide infra*).

The sticky nature typical of the hydrogel layer can also give problems with regard to any clothing worn by the wearer of the patch, in that the clothing may stick to the hydrogel layer and cause discomfort. This problem is addressed by the backing layer for the patch (*vide infra*).

The absorbent nature of the hydrogel layer means that is also permeable, as defined herein.

It is preferred that the hydrogel layer is breathable, particularly in conjunction with the backing layer being breathable and especially in conjunction with any further components also being breathable. Having breathable components leads to comfort in use advantages, enabling the skin the keep close to the temperature and humidity levels of its surroundings. In addition, the breathability can help maintain the natural microflora present on the skin and thereby avoid any changes in odour that could otherwise result from their perturbation.

It is preferred that the hydrogel layer is flexible, for comfort in use benefits. Indeed, it is preferred that all components of the patch are sufficient flexible to allow it to flex with the movement of the skin of the body to which it is attached.

The surface area of the hydrogel layer can be tailored to meet the needs of the specific skin site to which it is to be applied. In some preferred embodiments, this is from 40 to 70 cm², especially when it is intended for a single patch to be applied to each single axilla of an individual underarm. In other embodiments, especially where application of multiple patches to a given axilla is intended, the patches are preferably from 0.25 to 25 cm², more preferably 1 to 16 cm² and most preferably from 1 to 9 cm².

The thickness of the hydrogel layer may also be tailored to fit needs and may be from 1 micron to 1 cm; however, it is preferably from 0.5 to 6 mm and more preferably from 1 to 3 mm.

The water content of the hydrogel layer (immediately following manufacture of the patch) is preferably from 0 to 95%, more preferably from 0 to 60% and most preferably from 0 to 40% by weight, relative to the total weight of the hydrogel (including any water present).

The absorptive capacity of the hydrogel layer, as defined by its ability to absorb water at ambient temperature, is preferably from 100 or 200% by volume up to 400 or 500% by volume, based on the dry (i.e. zero water) volume of the hydrogel.

### Backing Layer

A backing layer for the hydrogel layer, present on its outer surface, is another essential component of the present invention. It main benefit, as mentioned above, is the avoidance of comfort-in-use issues that could otherwise result by the hydrogel layer sticking to any clothing worn by the wearer of the patch. Another benefit is that the backing layer may strengthen the associated hydrogel layer and thereby reduce damage to it, a particular problem in its most common location of use in the axillae.

It is preferred that the backing layer is flexible, for comfort in use reasons (*vide infra*).

It is preferred that the backing layer is breathable, for the same reasons as referred to with regard to the hydrogel reason.

It is preferred that the backing layer covers the majority, i.e. greater than 50% by area of the outer surface of the hydrogel layer. It is particularly preferred that the backing layer covers greater than 90% by area of the outer surface of the hydrogel layer.

In certain preferred embodiments, it is preferred that the backing layer overlaps the outer surface of the hydrogel layer and is sufficiently flexibility and overlapping to itself contact the skin when the patch is in operation. This feature is particularly advantageous when the backing layer has adhesion to the skin that is greater than that of the hydrogel layer.

The backing layer may comprise poly(alkoxyalkyl)acrylate (e.g. poly(2-hydroxyethyl)acrylate) or poly(alkoxyalkyl)methacrylate (e.g. poly(2-hydroxyethyl)methacrylate) or polyurethane. It is preferred that the backing material comprises a polyurethane elastomer and it is particularly preferred that this is the predominate material in the backing material. The polyurethane may be of an ester or ether based grade.

Suitable backing materials include the polyurethane elastomers available under the registered trademark ESTANE^{®} or Pellethane^{®}, both available from Lubrizol Corp.

An example of a suitable Estane^{®} is Estane^{®} 58315, an 85A aromatic polyether-based thermoplastic polyurethane (TPU) elastomer.

An example of a suitable Pellethane^{®} is Pellethane^{®} 5863-80A TPU, an aromatic polyether-based TPU elastomer.

### Pressure Sensitive Adhesive

The backing layer may additionally comprise a pressure sensitive adhesive (PSA), particularly on its inner surface. This is particularly advantageous when the hydrogel layer has a permeable membrane on its inner surface. When the hydrogel layer has a permeable membrane on its inner surface, this serves to separate it from the skin in use and may reduce its adhesion thereto. In such circumstances, giving the backing layer a PSA on its inner surface and giving it sufficiently flexibility and overlap to contact the skin when the patch is in operation can overcome this problem. Hence, the presence of a PSA on the inner surface of the backing layer, together with the presence of a permeable membrane on its inner surface and the backing layer having sufficiently flexibility and overlap to contact the skin when the patch is in operation is a desired combination of features.

Herein, a PSA is an adhesive that forms a bond between the substrates with which it is in contact when pressure is applied. Further, it can be detached without leaving traces.

When employed, the PSA is preferably an acrylic polymer, a polyvinyl ethyl ether or a triblock rubber copolymer of styrene with butadiene or isoprene, the triblock copolymer being formulated with oil. The acrylic polymers are preferably solvent-based, in particular water-based emulsions. Preferred acrylics are acrylic esters, such as 2-ethylhexyl acrylate and ethyl acrylate. The triblock rubber copolymers are preferably formulated with a tackifier as well as an oil.

Particularly suitable PSAs are DURO-TAK^{™} PSAs available from Henkel GmbH and LEVAGEL^{®} (a polyurethane PSA) available from Dow Corning.

### Tackifier

Herein, a tackifier is a chemical compound used to increase the "tack", i.e. stickiness of the surface of an adhesive. Preferred tackifiers are resins, in particular rosins and their derivatives; terpenes and modified terpenes; aliphatic, cycloaliphatic or aromatic resins (including C5 aliphatic resins and C5/C9 aliphatic/aromatic resins); hydrogenated hydrocarbon resins; terpene-phenol resins and mixtures thereof.

Tackifiers may be effectively employed in combination with the patches used in accordance with the present invention and particularly in combination with the hydrogel layer or any PSA so employed.

### Permeable Inner Membrane

Having a permeable inner membrane on the inner surface of the hydrogel layer is a feature of the first aspect of the present invention when the absorbent hydrogel layer is separated from direct contact with the skin. (*vide supra*). Such an inner membrane is not to be considered a feature of the hydrogel layer *per se*, but as an additional feature.

It is preferred that the permeable inner membrane is breathable, particularly in combination with the hydrogel layer and backing layer being breathable.

It is preferred that the permeable inner membrane is flexible, for comfort in use reasons. The permeable inner membrane is preferably a knitted, woven or non-woven structure forming a gauze, mesh or tulle.

In preferred embodiments, the permeable inner membrane is coated with a plastic porous film such as Telfa, which offers the additional benefit of allowing the patch to be more easily detached from the skin surface.

The permeable inner membrane does not occlude the skin. It is a thin film, which acts as a pliable barrier between the skin and the hydrogel layer and enhances comfort in wear.

Suitable materials for the permeable inner membrane include textiles such as polyester, rayon, cottons or mixtures such as polycotton. Other suitable materials are synthetic, porous polymer films, such as polyurethane, cellulose acetate, nitrocellulose, cellulose esters, polysulfone, polyether sulfone, polyacrylonitrile, polyamide, polyimide, polyethylene, polypropylene, polytetrafluoroethylene, polyvinylidene fluoride or polyvinylchloride.

### Extraction and Analysis

Once the patch absorbs the sweat, the malodourous volatile components are stable and available for subsequent extraction and analysis. The extraction may be performed by any suitable technique, including both traditional solvent extraction and more contemporary supercritical fluid extraction. The use of supercritical fluid extraction, in particular the use of supercritical carbon dioxide, is preferred because of its greater speed and ability to penetrate the hydrogel layer.

When supercritical carbon dioxide is used, this is preferably at a temperature of from 20°C to 50°C and at a pressure of from 100 to 300 Bar (from 10 to 30 MPa).

A further benefit of supercritical fluid extraction and the preferred versions of this referred to in the above paragraphs is that high extraction yields can be achieved, often approaching quantitative. In addition, the extraction can be achieved at relatively low temperature, a benefit of particular value in extraction of volatile malodourous materials as these can often be damaged by elevated temperatures.

The analysis of the extract from the hydrogel layer may be performed by any suitable technique. Preferred techniques are GC, LC, HPLC and SFC (supercritical fluid chromatography). SFC is a particularly preferred technique, especially when the extract is performed using supercritical fluid extraction.

### Examples

The following examples are provided to better illustrate the invention and are not to be considered limiting.
Figure 1 shows a cross-section through a patch (1) suitable for use in accordance with the invention.
Figure 2 shows how a patch may be located in the underarm region (axilla) and shows a top view of the patch (1).

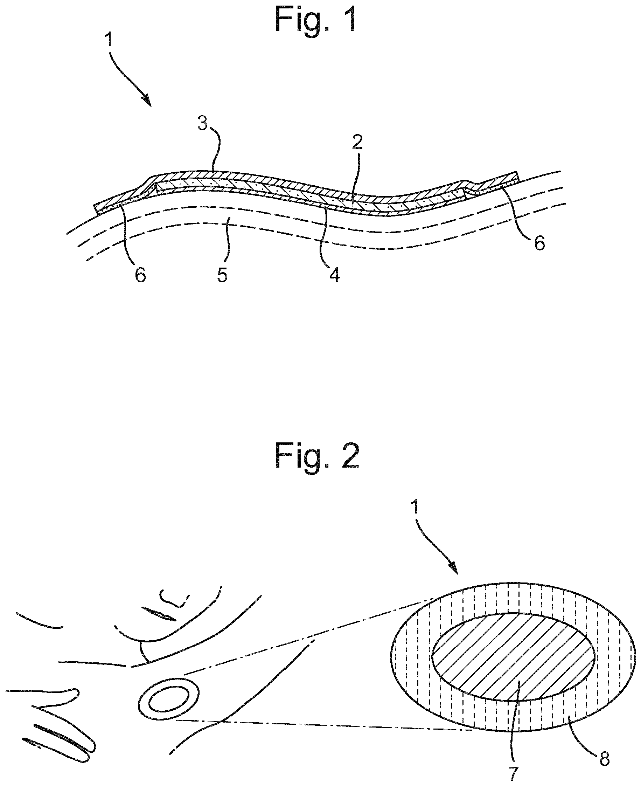

Figure 1 shows that the patch (1) comprises a hydrogel layer (2) sandwiched between a backing layer (3) and a permeable inner membrane (4) that contacts the skin (5). In this embodiment, each of the components is sufficiently flexible to allow the patch (1) as a whole to flex with the movement of the skin of the body to which it is attached.

Figure 1 further illustrates the backing layer (3) overlapping the hydrogel layer (2) and making contact with the skin (5) around the periphery of the hydrogel layer (2). The backing layer (3) adheres to the skin (5) in this peripheral region by means of a pressure sensitive adhesive (6) applied on the inner surface of the backing layer (3) around its periphery.

In Figure 2, the top view of the patch (1) illustrates a central region (7) and a peripheral region (8) of the patch. In the central region (7), the permeable inner membrane (4), hydrogel layer (2) and backing layer (3) lie one or top of the other. In the peripheral region (8), the backing layer (3) is stuck to the skin (5) by the pressure sensitive adhesive (6).

### Experiments

PolyAMPS hydrogel samples were synthesised using the following method. A batch of reaction mixture was prepared from 2-acrylamido-2-methylpropane sulfonic acid sodium salt (NaAMPS, 50% by weight in water, 46.4g in total), poly(ethylene glycol) diacrylate (PEGDA, Mₙ ~ 575 g mol⁻¹, 0.1077g), water (30.53g) and the photo-initiator Irgacure 1173 (2-hydroxy-2-methylpropiophenone, 0.1ml of a 10% aqueous solution). 3 ml samples were transferred to individual moulds and then photo-cured using high intensity UV radiation from a Light Hammer^{®} from Fusion UV Systems Corp.

In a first experiment, an artificial sweat sample comprising variety of malodourous volatile aliphatic acids (as often found in human perspiration) was applied to the surface of polyAMPS hydrogel samples (as prepared above). 0.5ml of the artificial sweat sample (comprising the aliphatic acids at 1 to 3 mmol dm⁻³) was applied and the samples placed on storage in heat-sealed aluminium pouches in an incubator at 25°C. Samples were removed at weekly intervals for analysis by Gas Chromatography (GC) using a Shimadzu GC2014 GC. The results are presented in Table 1 and are expressed as the average of 3 or 4 measurements for each of the indicated aliphatic acids vs. the amount originally applied. The results illustrate the excellent ability of the hydrogel to retain the volatile malodourous acids.

**Table 1**

| **Volatile acid added** | **Average Recovery (%) after...** | | | |
|---|---|---|---|---|
| | **1 week** | **2 weeks** | **3 weeks** | **4 weeks** |
| Acetic acid | 33.4 | 32.3 | 28.6 | 33.8 |
| Isobutyric acid | 89.1 | 89.6 | 81.1 | 72.1 |
| Butyric acid | 86.8 | 79.2 | 70.2 | 73.4 |
| Valeric acid | 71.3 | 65.2 | 71.6 | 58.0 |
| 4-methylvaleric acid | 49.2 | 41.5 | 39.7 | 32.3 |

In a second experiment, sweat patches according to the invention (comprising a polyAMPS hydrogel layer) were applied to the underarms of human volunteers. Following vigorous sporting activity, the patches were collected and extracted. Analysis of the extracts was by GC using a Shimadzu GC2014 GC. The retention time of the peaks in the worn sample were identified and compared with known retention times of volatile compounds tested. Linear regression analysis was used to determine the concentration based on pre-prepared calibration curves. GC analysis of the extracts revealed significant levels of acetic acid and propionic acid - both known malodourous components of human perspiration as typically exuded from the underarm regions. This is good evidence for the effectiveness of the method of the invention.

## Claims

1. A method of sampling volatile malodorous materials from the human skin, said method comprising the topical application of a patch comprising an absorbent hydrogel layer and a backing sheet adhering to it, the volatile malodourous materials being absorbed into the hydrogel layer and subsequently being extracted from it, the hydrogel layer being in direct with the skin or wherein the absorbent hydrogel layer is separated from direct contact with the skin by a permeable membrane, during the use of the patch.

2. A method according to claim 1, wherein the hydrogel layer comprises a hydrophilic polymer.

3. A method according to claim 2, wherein the hydrophilic polymer is poly(2-hydroxyethyl methacrylate) or a metal salt of poly(2-acrylo-2-methyl-1-propanesulfonic acid).

4. A method according to claim 3, wherein the hydrophilic polymer is a metal salt of poly(2-acrylo-2-methyl-1-propanesulfonic acid) crosslinked with poly(ethylene glycol) diacrylate.

5. A method according to any of preceding claims, wherein the backing sheet comprises a pressure sensitive adhesive.

6. A method according to claim 5, wherein the pressure sensitive adhesive comprises an acrylic polymer or a triblock copolymer of styrene with butadiene or isoprene, the block copolymer being formulated with oil.

7. A method according to any of preceding claims, wherein the hydrogel layer or pressure sensitive adhesive comprises a tackifier.

8. A method according to any of the preceding claims, wherein the extraction of the volatile materials from the hydrogel layer is performed using supercritical fluid technology.

9. A method according to any of the preceding claims, wherein the volatile malodourous materials are volatile fatty acids.

10. A patch for sampling volatile malodourous materials from the human skin comprising an absorbent hydrogel layer, a backing sheet adhering to it and a permeable inner membrane, which is held between the hydrogel layer and the skin when the patch is in use, wherein hydrogel layer comprises poly(2-hydroxyethylmethacrylate) or a salt of poly(2-acrylo-2-methyl-1-propanesulfonic acid).

11. A patch according to claim 10, wherein the hydrogel layer comprises poly(2 hydroxyethylmethacrylate) or a metal salt of poly(2-acrylo-2-methyl-1-propanesulfonic acid) crosslinked with polv(ethylene glycol) diacrylate.

12. A patch according to claim 10 or claim 11, wherein the backing sheet comprises a pressure sensitive adhesive.

13. A patch according to any of claims 10 to 12, wherein patch comprises a tackifier.

14. A patch according to any of claims 10 to 13, wherein all the components are permeable to air.

## Patentansprüche

1. Verfahren zur Probenahme flüchtiger übelriechender Materialien aus der menschlichen Haut, wobei das Verfahren die topische Verwendung eines Pflasters umfasst, das eine absorbierende Hydrogelschicht und eine daran haftende Abdeckfolie umfasst, wobei die flüchtigen übelriechenden Materialien in die Hydrogelschicht absorbiert und anschließend daraus extrahiert werden, wobei die Hydrogelschicht in direktem Kontakt mit der Haut steht oder wobei die absorbierende Hydrogelschicht von dem direkten Kontakt mit der Haut durch eine permeable Membran während der Verwendung des Pflasters separiert wird.

2. Verfahren nach Anspruch 1, wobei die Hydrogelschicht ein hydrophiles Polymer umfasst.

3. Verfahren nach Anspruch 2, wobei das hydrophile Polymer Poly(2-hydroxyethylmethacrylat) oder ein Metallsalz von Poly(2-acryl-2-methyl-1-propansulfonsäure) ist.

4. Verfahren nach Anspruch 3, wobei das hydrophile Polymer ein Metallsalz von Poly(2-acryl-2-methyl-1-propansulfonsäure), vernetzt mit Poly(ethylenglycol)diacrylat, ist.

5. Verfahren nach einem der vorhergehenden Ansprüche, wobei die Abdeckfolie einen Haftklebstoff umfasst.

6. Verfahren nach Anspruch 5, wobei der Haftklebstoff ein Acrylpolymer oder ein Triblockcopolymer von Styrol mit Butadien oder Isopren umfasst, wobei das Blockcopolymer mit Öl hergestellt ist.

7. Verfahren nach einem der vorhergehenden Ansprüche, wobei die Hydrogelschicht oder der Haftklebstoff einen Klebrigmacher umfasst.

8. Verfahren nach einem der vorhergehenden Ansprüche, wobei die Extraktion der flüchtigen Materialien aus der Hydrogelschicht unter Anwendung der überkritischen Fluidtechnologie durchgeführt wird.

9. Verfahren nach einem der vorhergehenden Ansprüche, wobei die flüchtigen übelriechenden Materialien flüchtige Fettsäuren darstellen.

10. Pflaster zur Probenahme flüchtiger übelriechender Materialien aus der menschlichen Haut, umfassend eine absorbierenden Hydrogelschicht, eine daran haftende Abdeckfolie und eine permeable innere Membran, die zwischen der Hydrogelschicht und der Haut gehalten ist, wenn das Pflaster in Gebrauch ist, wobei die Hydrogelschicht Poly(2-hydroxyethylmethacrylat) oder ein Salz von Poly(2-acryl-2-methyl-1-propansulfonsäure) umfasst.

11. Pflaster nach Anspruch 10, wobei die Hydrogelschicht Poly(2-hydroxyethylmethacrylat) oder ein Metallsalz von Poly(2-acryl-2-methyl-1-propansulfonsäure), vernetzt mit Poly(ethylenglycol)diacrylat, umfasst.

12. Pflaster nach Anspruch 10 oder Anspruch 11, wobei die Abdeckfolie einen Haftklebstoff umfasst.

13. Pflaster nach einem der Ansprüche 10 bis 12, wobei das Pflaster einen Klebrigmacher umfasst.

14. Pflaster nach einem der Ansprüche 10 bis 13, wobei alle Komponenten luftdurchlässig sind.

## Revendications

1. Procédé d'échantillonnage de matières malodorantes volatiles à partir de la peau humaine, ledit procédé comprenant l'application topique d'un patch comprenant une couche d'hydrogel absorbante et une feuille arrière adhérant à celle-ci, les matières malodorantes volatiles étant absorbées dans la couche d'hydrogel et étant ensuite extraites de celle-ci, la couche d'hydrogel étant en direct avec la peau ou dans lequel la couche d'hydrogel absorbante est séparée d'un contact direct avec la peau par une membrane perméable, pendant l'utilisation du patch.

2. Procédé selon la revendication 1, dans lequel la couche d'hydrogel comprend un polymère hydrophile.

3. Procédé selon la revendication 2, dans lequel le polymère hydrophile est le poly(méthacrylate de 2-hydroxyéthyle) ou un sel de métal de poly(acide 2-acrylo-2-méthyl-1-propanesulfonique).

4. Procédé selon la revendication 3, dans lequel le polymère hydrophile est un sel de métal de poly(acide 2-acrylo-2-méthyl-1-propane-sulfonique) réticulé avec du diacrylate de poly(éthylène glycol).

5. Procédé selon l'une quelconque des revendications précédentes, dans lequel la feuille arrière comprend un adhésif sensible à la pression.

6. Procédé selon la revendication 5, dans lequel l'adhésif sensible à la pression comprend un polymère acrylique ou un copolymère tri-séquencé de styrène avec du butadiène ou de l'isoprène, le copolymère séquencé étant formulé avec de l'huile.

7. Procédé selon l'une quelconque des revendications précédentes, dans lequel la couche d'hydrogel ou l'adhésif sensible à la pression comprend un agent collant.

8. Procédé selon l'une quelconque des revendications précédentes, dans lequel l'extraction des matières volatiles de la couche d'hydrogel est réalisée en utilisant une technologie au fluide supercritique.

9. Procédé selon l'une quelconque des revendications précédentes, dans lequel les matières malodorantes volatiles sont des acides gras volatiles.

10. Patch pour échantillonner des matières malodorantes volatiles à partir de la peau humaine comprenant une couche d'hydrogel absorbante, une feuille arrière adhérant à celle-ci et une membrane interne perméable, qui est maintenue entre la couche d'hydrogel et la peau lorsque le patch est utilisé, dans lequel la couche d'hydrogel comprend du poly(méthacrylate de 2-hydroxyéthyle) ou un sel de poly(acide 2-acrylo-2-méthyl-1-propanesulfonique).

11. Patch selon la revendication 10, dans lequel la couche d'hydrogel comprend du poly(méthacrylate de 2-hydroxyéthyle) ou un sel de métal de poly(acide 2-acrylo-2-méthyl-1-propanesulfonique) réticulé avec du diacrylate de poly(éthylène glycol).

12. Patch selon la revendication 10 ou revendication 11, dans lequel la feuille arrière comprend un adhésif sensible à la pression.

13. Patch selon l'une quelconque des revendications 10 à 12, dans lequel le patch comprend un agent collant.

14. Patch selon l'une quelconque des revendications 10 à 13, dans lequel tous les constituants sont perméables à l'air.
